# EUROPEAN PATENT APPLICATION

(11) **EP 1 181 912 A1**
(43) Date of publication of application: **27.02.2002**
(21) Application number: 99949485.9
(22) Date of filing: 27.09.1999
(51) Int. Cl.: A61F 9/00, G02C 1/00

(54) **DEVICE FOR CORRECTING LOW- AND MEDIUM-GRADE MYOPIA AND FOR PREVENTING ACCOMMODATION STRESS**

(30) Priority: 13.03.1999 RU 99105415
(71) Applicant: Kugoeva, Ekaterina Emmanuilovna, Moscow, 121002 (RU)
(72) Inventor: Kugoeva, Ekaterina Emmanuilovna, Moscow, 121002 (RU)
(74) Representative: W.P. Thompson & Co.
(86) International application number: RU9900354
(87) International publication number: WO0054710

(57) **Abstract**

The invention relates to medicine, namely, to ophthalmology and can find application for treating and preventing progressive myopia, and also for relieving accommodation stress in individuals with progressive myopia. The attainable technical results resides in relieving accommodation by a combined use of myopia-correcting spectacle lenses at the top and zero correction of vision at the bottom as well as in providing favorable functional conditions for the eye of a myopic individual. The device comprises a spectacle frame whose lens holders carry spectacle lenses capable of correcting far vision. The lower border of the spectacle lenses is disposed at the level of the edge of the lower eyelid with the user's gaze directed straight ahead. The bridge of the spectacle frame lies at the level of the lower border of the lenses. The device is aimed at correcting low- and medium-degree myopia, wherein the position of the far point provides good near vision without correction. Use of the device by myope-eyed individuals provides for conditions for an adequate correction of far vision accompanied with relieving near vision. Use of special spectacles for myope-eyed individuals is necessary in constantly changing the direction of vision from far to near, and in alternately viewing at objects situated at a long and short distances from user's eyes provided he/she has a good near vision.

## Description

### Technical Field

This invention relates in general to medicine, more specifically, to ophthalmology and can find application for treating and preventing progressive myopia, as well as for relieving accommodation stress in patients suffering from progressive myopia.

### Background Art

A concept of providing optimum conditions for normal functioning of the ciliary muscle has long been known and is know used in bifocal spectacles adapted for correcting far and near vision under conditions of constant changing the direction of far vision for a final distance before eyes. Bifocal lenses are characterized by combining in the same frame the lenses of different focal power, namely, the top lens being for far vision, and the top lens, for near vision (cf. the textbook "Optometry" by Yu.Z.Rosenblum, 1996, p.151 (in Russian). Bifocal lenses are used for correcting various kinds of ametropy (i.e., myopia and hypermetropy) under conditions of presbiopy (age-dependent changes of accommodation characterized by the fact that the near point of vision is removed away from the eye). In ophthalmological practice there are cases where for the bottom lens portion is in fact the "planum" grade one. However, such spectacles restrict patient's visual field and are hardly tolerable by patients due to too a small area of optical additive. Hence traditional bifocal spectacles may be regarded as an analog to the present invention.

The closest analog to the present invention is a device appearing as bifocal spheroprismatic spectacles (cf. op. cit., p.152) aimed at correcting various-degree myopia. The device comprises lenses mounted in the same frame and differing in focal power, i.e., the top lens for correcting far vision (the submaximum lens power depending on the degree of myopia, and an optically neutral additive appearing as a lens of +2.25 diopter with a prismatic component).

A material disadvantage inherent in the spheroprismatic spectacles and restricting their practical use resides in inconvenient use of an optical field of vision in near vision due to too a small area of optical lens which narrows the near space being corrected and causes the sense of discomfort in patients.

Similar negative attitude to the use of additional correction at the bottom arises in patients using standard bifocal lenses. Insofar as modem production process techniques of bifocal lenses involve turning a spectacle lens for short-distance vision from a monolithic optical-glass blank for a long-distance spectacle lens, too small an area of the bottom lens is predetermined and hence becomes inevitable. Such bifocal lenses are convenient when worn constantly, while walking inclusive, but they limit the working space in near-vision during a prolonged working at a short distance (e.g., while sitting at a writing-desk).

### Summary of the Invention

One of the most important concepts of all theories of myopia progression in children and adolescents is the accommodation stress phenomenon leading to a persistent accommodation spasm which is likely to give rise first to false myopia and then to true myopia which progresses constantly. Correction of far vision in myopic patients is necessary for the pupils during the whole period of education, that is, daily attending school auditoriums and looking at the blackboard at a long-distance to recognize information thereon and transfer it to the copy-books. However, at the instant of writing the information in the copy-book the far vision correcting spectacles occurs to be too high-power for myope-eyed individuals, thus causing the ciliary muscle of the eye to overstrain.

With spheroprismatic spectacles near vision is relieved due to an optical neutralizing additive, though visual inconvenience remains due to a too narrow field of near vision.

A technical result attainable by the present invention resides in accommodation relieving when using spectacle lenses for myopia correction at the top and zero visual correction at the bottom, as well as in providing favorable conditions for the eye of a myopic patient to function normally.

The essence of the invention consists in attaining said technical result due to the use of a device for low- and medium-degree myopia and for preventing accommodation stress, characterized in that it appears as spectacles for both far and near vision, wherein the spectacle lens is adapted to provide correction of far vision alone, and the lower border of the spectacle lens lies at the level of the edge of the lower eyelids, with the patient gazing straight ahead and the bridge of the spectacle frame disposed at the level of the lower border of lenses.

### Brief Description of the Drawings

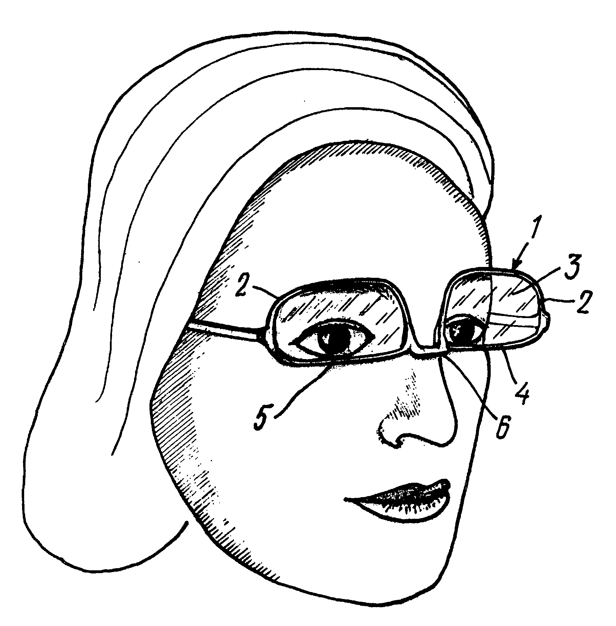

The accompanying drawing illustrates schematically the device, according to the invention.

### Best Method of Carrying Out the Invention

The device for correcting low- and medium-degree myopia and for preventing accommodation stress comprises a spectacle frame 1 whose lens holders 2 carry spectacle lenses 3 capable of correcting far vision. A lower border 4 of the spectacle lenses 3 is disposed at the level of the edge of a lower eyelid 5, with the patient gazing straight ahead. A bridge 6 of the spectacle frame lies at the level of the lower border 4 of the lenses 3.

Used as the spectacle frame may be a standard frame, wherein the lower portion of the lens holder is to be cut off up to a level corresponding to the position assumed by the user's lower eyelid when he/she gazes straight ahead. The size of a spectacle lens for correcting far vision should correspond to that of the lens holder. The bridge of the spectacle frame is disposed at the level of the lower border of the lenses. The frame together with the spectacles may be manufactured by press-forming.

### Industrial Applicability

The device is adapted for correcting low- and medium-degree myopia, wherein the position of the far point, i.e., the degree of shortsightedness provides good near vision without correction. Hence when using the device of the present invention, conditions are provided in myope-eyed individuals for adequately correcting far vision, accompanied with relieving near vision.

Use of special spectacles for myope-eyed individuals is necessary in constantly changing the direction of vision from far to near, and in alternately viewing at objects situated at a long and short distances from user's eyes provided he/she has a good near vision.

High-degree myopia and to some extent medium-degree myopia requires mandatory correction of near vision for maintaining at least a 20-cm working distance of vision to suit anthropometric characteristics of human body, whereby patients affected by high-degree myopia need standard bifocal spectacles.

### Example 1

Male patient R., 11, has been under regular observation for three recent years, the diagnosis being one of low-degree non-progressing myopia of both eyes. Three years ago there had been detected constant deterioration of far vision. The diagnosis of progressive myopia was confirmed against the background of a spasm of accommodation. It is known from the anamnesis that the child patient undergoes very high visual strain both at school and at home for as long as 1.5 years with the result that the spectacle lenses should be changed for higher-power ones every half a year. The spectacles prescribed for far vision were used by the patient during the school sessions for work under a near-vision conditions (while writing or reading). After prolonged atropinization and relieving the spasm of accommodation a 1.5 diopter myopia was detected.

The patient was provided with spectacles for use at school, having correcting lenses at the top and no correcting lenses at the bottom, according to the invention. Far-vision spectacles provided with a complete set of lenses were be used by the patient rather rarely, only for watching entertainment performances. The degree of myopia displayed no progression with a one-year period which is indicative of stabilization of the process.

### Example 2

Female patient K., 41, sought medical advice for complaints of asthenopic phenomena, permanent headache, poor tolerance of the spectacles. Upon examination a 3.75 diopter myopia in both eyes was detected. Regular work of the patient involved constant visual strain; the patient made practically permanent use of the spectacles with 3.75 diopter correcting lenses. Bifocal correcting lenses - 3.5 diopter at the top and the "planum" grade at the bottom were very inconvenient and therefore found no use by the patient.

There were custom-made for the patient the spectacles, according to the invention. Any asthenopic complaints on the part of the patient disappeared within a month without taking any additional measures whatever.

Spectacles for correcting low- and medium-degree myopia having a correction-free space for near vision may be applied for correcting both progressive myopia in children and adolescents and stationary myopia in presbyopic-age patients. In the former case, near vision strain in pupils during class session contributes to preventing the spasm of accommodation and precludes realizing the principal myopia progressing mechanism. In the latter case, when presbyopy develops prematurely at the age of 35-40, relieving near vision eliminates the strain of accommodation and patient's asthenopic complaints, thereby adding to patients' working capacity. Hence the spectacles of the present invention can find widespread application in myopic-refraction individuals within the various age brackets. The high position of the lenses in the frame contributes to an adequate correction of far vision and renders correction of near vision unnecessary when patient shifts his/her gaze downwards for a short distance. Thereby the ciliary muscle of the eye receives favorable conditions for spontaneous independent without accommodation stress.

## Claims

1. A device for correcting low- and medium-degree myopia and for preventing accommodation stress of a user, **CHARACTERIZED in that** it appears as spectacles intended for both far and near vision, wherein each spectacle lens is adapted to provide correction of far vision only, and the lower border of each spectacle lens is disposed at the level of the edge of the lower eyelid of the user in case of gazing straight ahead, while the bridge of the spectacle frame is disposed at the level of the lower border of the lenses.
